(19) Europäisches Patentamt
European Patent Office
Office européen des brevets

(11) Numéro de publication: **0 220 104 B1**

(12) **FASCICULE DE BREVET EUROPEEN**

(45) Date de publication de fascicule du brevet: **15.04.92**

(21) Numéro de dépôt: **86402181.1**

(22) Date de dépôt: **03.10.86**

(51) Int. Cl.5: **C07D 215/26**, C07D 405/12, C07D 319/18, C07C 255/32, C07C 255/36, C07D 317/60, C07D 417/12, C07D 413/12, A61K 31/47

# (54) Dérivés d'amino-5 pentanenitrile, leurs procédés de préparation et les compositions pharmaceutiques qui les contiennent.

(30) Priorité: **04.10.85 FR 8514756**

(43) Date de publication de la demande: **29.04.87 Bulletin 87/18**

(45) Mention de la délivrance du brevet: **15.04.92 Bulletin 92/16**

(84) Etats contractants désignés: **AT BE CH DE FR GB IT LI LU NL SE**

(56) Documents cités: **EP-A- 0 064 158** **FR-M- 2 663**

(73) Titulaire: **ADIR ET COMPAGNIE**
**1 rue Carle Hébert**
**F-92415 Courbevoie Cédex(FR)**

(72) Inventeur: **Regnier, Gilbert**
**27 avenue du Plessis**
**F-92290 Chatenay Malabry(FR)**
Inventeur: **Gargouil, Yves-Michel**
**38 rue Michel Ange**
**F-75016 Paris(FR)**
Inventeur: **Vilaine, Jean-Paul**
**5 rue Arthur Rand**
**F-92350 Le Plessis Robinson(FR)**

(74) Mandataire: **Reverbori, Marcelle ADIR ET COMPAGNIE et al**
**1, rue Carle Hébert**
**F-92415 Courbevoie Cédex(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention a pour objet de nouveaux dérivés d'amino-5 pentanenitrile, leurs procédés de préparation et les compositions pharmaceutiques les renfermant.

Elle concerne particulièrement les dérivés d'amino-5 pentanenitrile de formule générale I :

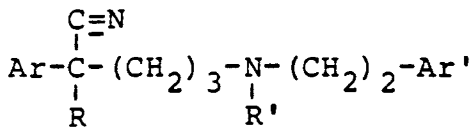

(I)

dans laquelle :

- Ar représente un radical de formule :

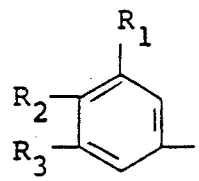

dans laquelle :

- $R_1$ représente un atome d'hydrogène ou d'halogène, un radical trifluorométhyle ou un radical alcoxy contenant de 1 à 5 atomes de carbone ; et
- $R_2$ et $R_3$, identiques ou différents, représentent chacun un atome d'hydrogène ou un radical alcoxy contenant de 1 à 5 atomes de carbone, ou
- $R_2$ et $R_3$ représentent ensemble un radical : -O-CH = CH-O- ou $-O-CH_2-O-$, ou
- $R_2$ et $R_3$ forment ensemble, avec le radical phényle auquel ils sont liés un radical benzoxazolyle, benzothiazolyle, benzothiadiazolyle ou quinoléïnyle,
- R est un radical alcoyle contenant de 3 à 15 atomes de carbone en chaîne droite ou ramifiée, ou un radical de formule :

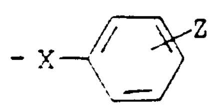

dans laquelle :

- X représente S ou $SO_2$ et Z représente un atome d'hydrogène, de chlore ou un radical alcoyle ou alcoxy ayant chacun de 1 à 5 atomes de carbone,
- R' représente un radical alcoyle contenant de 1 à 5 atomes de carbone ; et
- Ar' représente :

un radical de formule :

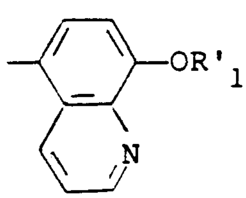

ou

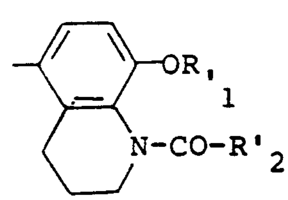

dans lesquelles :

- $R'_1$ représente un radical alcoyle contenant de 1 à 5 atomes de carbone en chaîne droite ou ramifiée, et
- $R'_2$ représente un atome d'hydrogène ou, un radical alcoyle contenant de 1 à 4 atomes de carbone

en chaîne droite ou ramifiée.

L'état antérieur de la technique dans ce domaine est illustré notamment par le brevet français de Médicament n° 2663 M et la demande de brevet européen n° 0064.158, qui ont pour objet des phénylacétonitriles de formule :

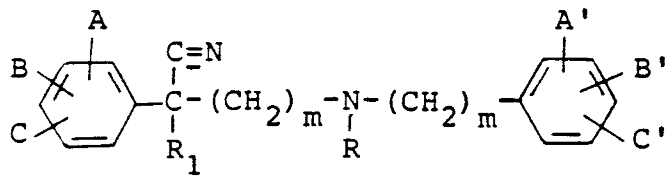

dans laquelle :

- m et n sont des nombres entiers de 2 à 5,
- R et $R_1$ représentent, entre autres, un radical alcoyle inférieur, ou un radical phényle,
- A, B, C, A', B', et C' sont des substituants classiques dont hydrogène, halogène, alcoyle ou alcoxy inférieurs ou méthylène dioxy ;

Ces phénylacétonitriles, dont le produit commercial le plus représentatif est le vérapamil, ont une activité spasmolytique, augmentent l'irrigation coronaire et ont une activité dilatatrice des vaisseaux coronaires.

Par ailleurs, il est connu que le vérapamil est une substance cardiotrope ayant des effets inotropes négatifs et surtout induisant un allongement de l'espace PR de l'électrocardiogramme traduisant ainsi une diminution de la conduction auriculo-ventriculaire et entrainant, en utilisation clinique, des effets secondaires néfastes tel que, par exemple, la fibrillation.

Les produits de la présente invention, et en particulier le dérivé objet de l'exemple 1 ci-après décrit, ne présentent pas ces inconvénients car ils se démarquent du vérapamil sur le plan pharmacologique par une plus grande affinité pour les canaux calciques pouvant être 20 fois celle du vérapamil, une affinité pour la calmoduline cohérente avec l'effet biologique et une sélectivité plus grande en faveur des vaisseaux ce qui permet de réguler l'hypertension artérielle en agissant préférentiellement sur les vaisseaux et non sur le myocarde évitant ainsi les phénomènes secondaires néfastes et notamment les risques de fibrillation.

La présente invention a également pour objet le procédé de préparation des dérivés de formule générale I caractérisé en ce que l'on fait réagir :

- un composé halogéné de formule générale II :

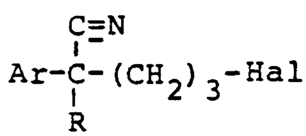

(II)

dans laquelle :

- Ar et R ont les significations précitées, et
- Hal représente un atome d'halogène, tel que par exemple un atome de chlore ou de brome,
- avec une amine de formule générale III :

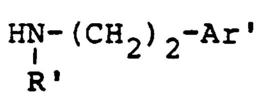

(III)

dans laquelle :

- R' et Ar' ont les significations précédemment définies. Il est particulièrement avantageux d'effectuer cette réaction dans un solvant approprié tel que, par exemple, l'acétonitrile, la méthyléthyl cétone et les alcools miscibles à l'eau, de bas point d'ébullition, en opérant à une température comprise entre 60 et 100 ° C.

La présente invention a aussi pour objet le procédé de préparation des dérivés de formule générale I caractérisé en ce que l'on fait réagir :

- un composé de formule générale IV :

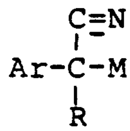

(IV)

dans laquelle :

- Ar et R ont les significations précédemment définies, et
- M représente un atome de sodium ou de lithium ;
- avec un dérivé halogéné de formule générale V :

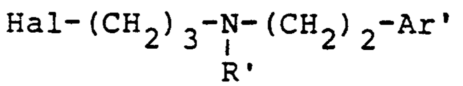

(V)

dans laquelle :

- R' et Ar' sont tels que précédemment définis, et
- Hal représente un atome d'halogène, tel que par exemple un atome de chlore ou de brome.

Une telle réaction s'effectue d'une façon particulièrement adéquate dans un solvant approprié tel que par exemple, le tétrahydrofuranne ou le toluène, à une température comprise entre 60 et 110 ° C.

La présente invention inclut également le procédé de préparation des dérivés de formule générale I dans le cas ou Ar' représente

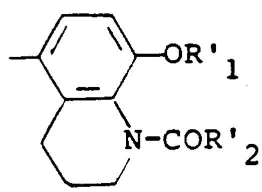

c'est-à-dire les dérivés de formule générale I' :

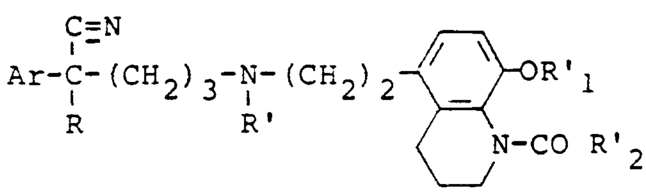

(I')

dans laquelle Ar, R, R', $R'_1$ et $R'_2$ ont les significations précédemment définies, caractérisé en ce que :

- l'on hydrogène le composé de formule générale VI :

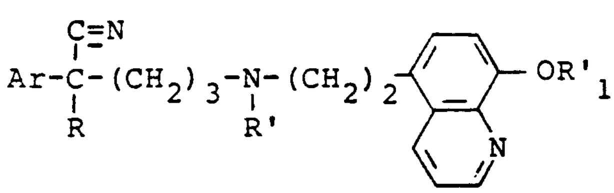

(VI)

dans laquelle Ar, R, R' et $R'_1$ ont les significations précédemment définies et

- l'on acyle le composé ainsi obtenu de formule générale VII:

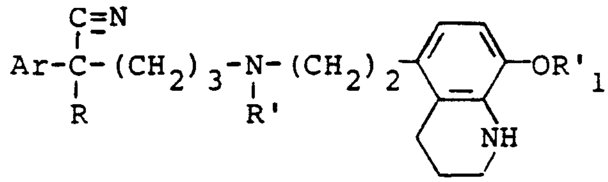

(VII)

dans laquelle Ar, R, R' et $R'_1$ sont tels que précédemment définis, avec un chlorure d'acide de formule générale VIII :

$R'_2$ CO Cl (VIII)

dans laquelle $R'_2$ a la signification énoncée précédemment.

Le composé (VI) qui n'est autre que le dérivé de formule générale I dans laquelle Ar' représente :

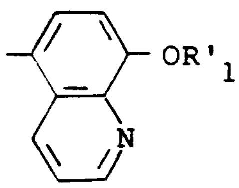

a été préparé selon l'une des deux méthodes de préparation énoncées ci-dessus.

L'hydrogénation du composé (VI) est réalisée, de façon particulièrement adéquate, par l'hydrogène en présence de nickel sous une pression de 50 à 110 atmosphères, dans un alcool à bas point d'ébullition, à une température voisine de 90 ° C, ou au moyen d'un hydrure tel que $BH_3CN$ Na dans le tétrahydrofuranne à température ambiante.

Il est avantageux de réaliser l'acylation du composé VII par $R'_2$CO Cl dans le tétrahydrofuranne, en présence de triéthylamine, ou dans le cas ou $R'_2$ est hydrogène avec le mélange HCOOH 99% diméthylformamide.

Les dérivés (I) préparés selon les procédés énoncés ci-dessus peuvent être purifiés soit par chromatographie flash sur colonne $SiO_2$(230-400 mesh) sous 0,5-0,8 bar $N_2$, en employant les systèmes AcOEt ou $CH_2Cl_2$-MeOH (95-5) ou benzène - MeOH (95-5), soit par recristallisation sous forme de sels. Les nouveaux dérivés (I) ainsi obtenus peuvent être transformés en sels d'addition avec les acides, sels qui font, à ce titre, partie de la présente invention. Comme acides, utilisables pour la formation de ces sels, on peut citer par exemple, dans la série minérale : les acides chlorhydrique, bromhydrique, sulfurique, phosphorique et dans la série organique, les acides acétique, propionique, maléïque, fumarique, tartrique, citrique, oxalique, benzoïque, méthane sulfonique et iséthionique.

Les matières premières nécessaires à la préparation des dérivés I ont elles mêmes été préparées selon les techniques décrites dans la littérature. Ainsi :

- les composés de formule

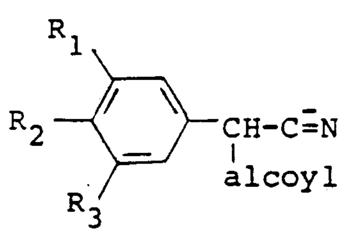

ont été préparés selon CARLSON et Coll. Helv. 46,2271 (1983);

- les composés de formule :

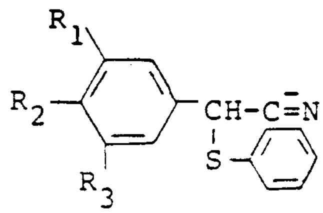

ont été préparés selon E. MARCHAND, G. MOREL et A. FOUCAUD Synthesis (1978), 360-361 et purifiés par chromatographie flash ;

- les composés de formule

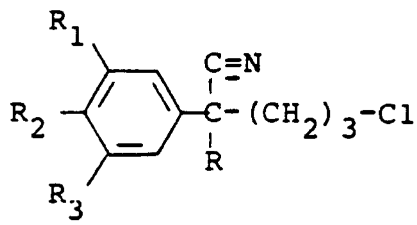

ont été préparés selon le brevet belge N° 704,190 du 22 septembre 1967 (KNOLL), et purifiés à l'état d'huiles par chromatographie flash ; et

- les composés de formule :

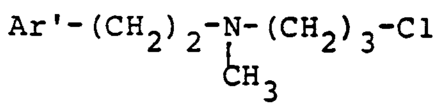

ont été préparés selon RAMUZ - Arzn. Forsh. 28 (II), 2049-2050, (1978), et purifiés par distillation sous pression réduite, ou chromatographie flash.

Les caractéristiques des matières premières de formule :

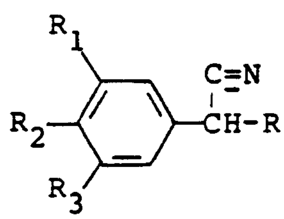

utilisées pour préparer les composés objet des exemples ci-après décrits sont récapitulées dans le tableau ci-après :

## CARACTERISTIQUES DES COMPOSES DE FORMULE :

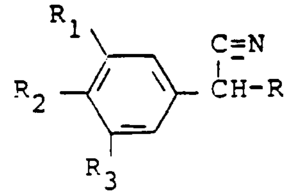

| COMPOSE REFERENCE | $R_1$ | $R_2$ | $R_3$ | R | CONSTANTE PHYSIQUE |
|---|---|---|---|---|---|
| a | $-OCH_3$ | H | H | 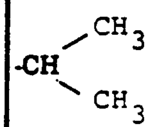 | Eb/1066 Pa (8 mmHg) =106-110°C $n_{24}^{D}=1{,}5135$ |
| b | $-OCH_3$ | H | H | $-(CH_2)_{11}-CH_3$ | Eb/400 Pa (0,3 mmHg) =160-190°C $n_{24}^{D}=1{,}4910$ |
| c | $-OCH_3$ | H | H | 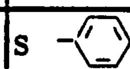 | PF : 82°C |
| d | -CF3 | H | H | 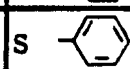 | Huile |
| e | $-OCH_3$ | $-OCH_3$ | H | 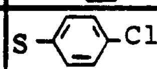 | Huile |
| f | $-OCH_3$ | $-OCH_3$ | H | 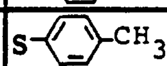 | Huile |
| g * | $-OCH_3$ | $-OCH_3$ | H | 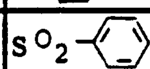 | PF : 126°C |
| h | H | | | 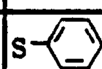 | Huile |
| i | H | | | 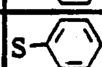 | PF : 72°C |
| j | H | | | 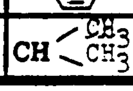 | Huile |

* **Le dérivé (g) a été préparé par oxydation du dérivé corespondant dans lequel R= -S 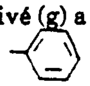 avec le pyridinium chlorochromate dans $CH_2Cl_2$.**

Les caractéristiques des matières premières (VI) utilisées dans les exemples ci-après décrits sont récapitulées dans le tableau suivant:

## CARACTERISTIQUES DES COMPOSES DE FORMULE :

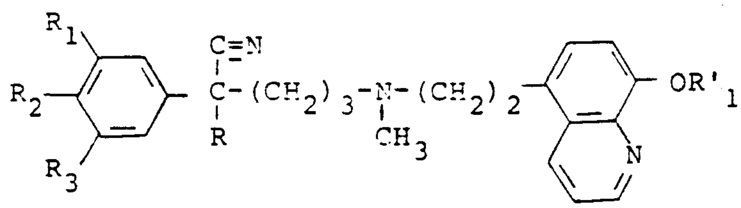

$R'_1=CH_3$

| $R_1$ | $R_2$ | $R_3$ | R | FORME ISOLEE | PF |
|---|---|---|---|---|---|
| H | $-OCH_3$ | $-OCH_3$ | $-CH{<}^{CH_3}_{CH_3}$ | dichlorhydrate | 200°C |
| H | | | $-CH{<}^{CH_3}_{CH_3}$ | dichlorhydrate | 202°C |
| H | $-OCH_3$ | $-OCH_3$ | $SO_2$ 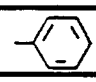 | dichlorhydrate | 188°C |
| H | $-OCH_3$ | $-OCH_3$ | S 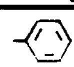 | base | Huile |
| H | | H | S 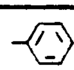 | base | Huile |
| $-CF_3$ | H | H | S 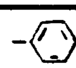 | base | Huile |
| H | | | S 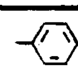 | base | Huile |
| H | $-OCH_3$ | $-OCH_3$ | S 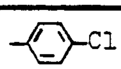 | base | Huile |
| H | $-OCH_3$ | $-OCH_3$ | S 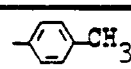 | base | Huile |
| $-OCH_3$ | H | H | $-CH{<}^{CH_3}_{CH_3}$ | base | Huile |
| $-OCH_3$ | H | H | $-(CH_2)_{11}-CH_3$ | base | Huile |
| $-OCH_3$ | H | H | S 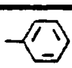 | base | Huile |

Les dérivés de formule générale I et leurs sels physiologiquement tolérables possèdent des propriétés pharmacologiques et thérapeutiques intéressantes, notamment des propriétés antagonistes des mouvements intracellulaires du calcium.

L'étude pharmacologique des composés de l'invention a été réalisée :

- in vitro par des études sur les organes isolés de rat, et en ce qui concerne la fixation à la calmoduline et
- in vivo, chez le chien en comparaison avec les propriétés du vérapamil, agent anti-calcique bien connu, pris comme produit de référence.

Les modes opératoires et les résultats obtenus font l'objet des exemples pharmacologiques inclus ci-après.

Les essais pharmacologiques in vitro ont montré que ces composés sont de puissants modulateurs des mouvements intracellulaires et transmembranaires du calcium. Certaines activités cellulaires des muscles lisses ou striés, notamment leur contractilité, sont liées à la concentration intracytoplasmique du calcium, et la perturbation de cette concentration a pu être mise en évidence dans certaines affections faisant intervenir la contractilité musculaire dont notamment l'angor, l'hypertension artérielle, l'asthme, la migraine et les spasmes oesophagiens.

Le calcium a également un rôle dans la régulation du métabolisme cellulaire, en particulier mitochondrial, lequel métabolisme est perturbé dans les pathologies comme l'ischémie cardiaque ou cérébrale.
Les propriétés pharmacologiques des dérivés de l'invention permettent donc leur application dans le traitement des maladies nécessitant des modulateurs de mouvements transmembranaires et intracellulaires du calcium et en particulier dans le traitement de l'hypertension, de l'angor, de l'asthme, des spasmes oesophagiens, de la migraine ou de l'ischémie myocardique et cérébrale.

La présente invention a également pour objet les compositions pharmaceutiques contenant comme principe actif un dérivé de formule générale I ou un de ses sels physiologiquement tolérables, mélangé ou associé à un excipient pharmaceutique approprié.
Les compositions pharmaceutiques ainsi obtenues sont présentées avantageusement sous des formes diverses telles que par exemple, comprimés, dragées, gélules, glossettes ou préparations galéniques appropriées pour une administration sublinguale, suppositoires ou solutions injectables ou buvables, et être administrées par voie orale, rectale ou parentérale. La posologie peut varier largement en fonction de l'âge et du poids du patient, de la nature et de la sévérité de l'affection à traiter ainsi que de la voie d'administration. D'une manière générale, en thérapeutique humaine, par voie IV ou per os, la posologie unitaire s'échellonnera entre 10 et 200 mg et la posologie journalière entre 30 et 600 mg.

Les exemples suivants, donnés à titre non limitatif, illustrent l'invention. Les points de fusion, sauf mention contraire, sont déterminés à la platine chauffante de Kofler.

EXEMPLE 1 :

(méthoxy-3 phényl)-2 phénylthio-2{N-[(acétyl-1 méthoxy-8 tétrahydro-1,2,3,4 quinoléinyl-5) éthyl] N-méthylamino}5 pentanenitrile

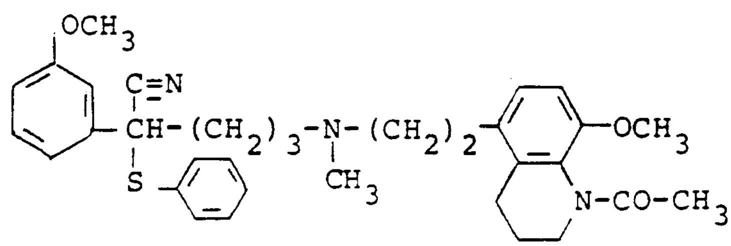

On chauffe pendant 20 heures à reflux une solution de 3,3 g de (méthoxy-3 phényl)-2 phénylthio-2 chloro-5 pentanenitrile fondant à 50-52° C et de 5 g d'acétyl-1 méthoxy-8 (méthylamino éthyl)-5 tétrahydro-1,2,3,4 quinoléine (huile) dans 250 ml d'acétonitrile en présence de 0,2 g d'iodure de sodium. Lorsque la réaction est terminée, on refroidit et essore les cristaux de chlorhydrate de l'amine de départ puis évapore le filtrat à sec. Le résidu est dissous dans l'éther et la solution lavée à l'eau. Après évaporation de la couche organique, la base huileuse obtenue est transformée en fumarate au sein de l'éthanol, on obtient, sous forme de sel amorphe, 3 g de fumarate de (méthoxy-3 phényl)-2 phénylthio-2{N-[(acétyl-1 méthoxy-8 tétrahydro-1,2,3,4 quinoléïnyl)-5 éthyl] N-méthylamino}-5 pentanenitrile. L'amine de départ a été préparée par débenzylation avec $H_2$-$Pd(OH)_2$ sous 6 atm $H_2$ de l'acétyl-1 méthoxy-8 (N-méthyl N-benzylamino éthyl)-5 tétrahydro-1,2,3,4 quinoléine elle-même preparée par acétylation au moyen de $CH_3COCl$-$(Et)_3N$ dans le tétrahydrofuranne, de la méthoxy-8 (N-méthyl N-benzylamino éthyl)-5 tétrahydroquinoléine préparée par action de la N-méthyl benzylamine sur le chlorhydrate de méthoxy-8 chloréthyl-5 tétrahydro-1,2,3,4 quinoléine dans l'éthanol à reflux, lui-même préparé par chloruration avec un excès de $SO\ Cl_2$ du chlorhydrate du dérivé hydroxyéthylé correspondant, lui-même préparé par hydrogénation avec $H_2$/Ni sous 100 atm de l'hydroxyéthyl-5 méthoxy-8 quinoléine fondant à 134° C.
Le (méthoxy-3 phényl)-2 phénylthio-2{N-[(acétyl-1 méthoxy-8 tétrahydro-1,2,3,4 quinoléinyl-5) éthyl] N-méthylamino}-5 pentanenitrile a également été préparé selon le procédé décrit dans l'exemple 2 suivant.

EXEMPLE 2 :

(benzodioxinyl-6)-2 isopropyl-2 {N-[(méthoxy-8 quinoléinyl-5) éthyl] N-méthylamino}-5 pentanenitrile

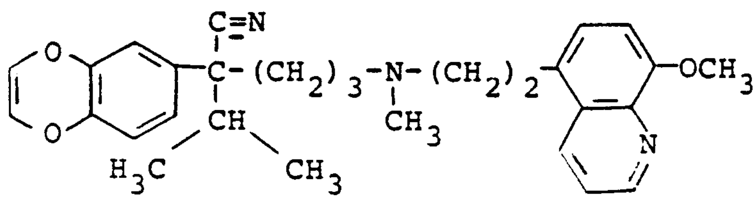

A partir de 75 ml de $NH_3$ liquide et de 9,9 g de sodium, on prépare l'amidure selon la méthode conventionnelle. A la suspension obtenue, on ajoute 7,7 g d'isopropyl (benzodioxinyl-6) acétonitrile. On laisse évaporer l'ammoniac et ajoute 75 ml de toluène anhydre. Dans la suspension obtenue, on coule une solution de 12,5 g de [(N-chloro-3 propyl N-méthyl) amino éthyl]-5 méthoxy-8 quinoléine (huile) dans 20 ml de toluène anhydre, on chauffe pendant 8 heures au reflux, refroidit, reprend à l'eau. La couche organique décantée est évaporée et le résidu est chromatographié sur 330 g de silice (éluant $CH_2Cl_2$-MeOH 95-5). Pression 0,5 atm de $N_2$ ; on obtient 10 g de base huileuse dont on prépare le chlorhydrate (amorphe) au sein de l'éthanol avec HCl dans EtOH ;
On obtient ainsi le dichlorhydrate hémihydrate de (benzodioxinyl-6)-2 isopropyl-2{N-[(méthoxy-8 quinoléinyl-5) éthyl] N-méthylamino}-5 pentanenitrile, fondant instantanément à 202° C. Ce composé a également été préparé selon la méthode objet de l'exemple 1.

EXEMPLES 3 à 24 :

Les dérivés suivants ont été préparés selon méthodes décrites dans les exemples 1 et 2.

3) (diméthoxy-3,4 phényl)-2 isopropyl-2{N-[(méthoxy-8 quinoléinyl-5)éthyl] N-méthylamino}-5 pentanenitrile, P.F. du dichlorhydrate : 200° C (éthanol anhydre).

4) (diméthoxy-3,4 phényl)-2 benzènesulfonyl-2{N-[(méthoxy-8 quinoléinyl-5) éthyl] N-méthylamino}-5 pentanenitrile, P.F. du dichlorhydrate : 188° C (éthanol anhydre).

5) (diméthoxy-3,4 phényl)-2 isopropyl-2{N[(acétyl-1 méthoxy-8 tétrahydro-1,2,3,4 quinoléinyl-5) éthyl] N-méthylamino}-5 pentanenitrile, P.F. du chlorhydrate : 198° C (éthanol anhydre).

6) (benzodioxinyl-6)-2 isopropyl-2{N-[(acétyl-1 méthoxy-8 tétrahydro-1,2,3,4 quinoléinyl-5) éthyl] N-méthylamino}-5 pentanenitrile.

7) (diméthoxy-3,4 phényl)-2 phénylthio-2{N-[(acétyl-1 méthoxy-8 tétrahydro-1,2,3,4 quinoléinyl-5)éthyl] N-méthylamino}-5 pentanenitrile.

8) (diméthoxy-3,4 phényl)-2 phénylthio-2{N-[(acétyl-1 méthoxy-8 tétrahydro-1,2,3,4 quinoléinyl-5) éthyl] N-méthylamino}-5 pentanenitrile.

9) (benzodioxinyl-6)-2 phénylthio-2{N-[(acétyl-1 méthoxy-8 tétrahydro-1,2,3,4 quinoléinyl-5) éthyl] N-méthylamino}-5 pentanenitrile.

10) (trifluorométhyl-3 phényl)-2 phénylthio-2{N-[(acétyl-1 méthoxy-8 tétrahydro-1,2,3,4 quinoléinyl-5 éthyl] N-méthylamino}-5 pentanenitrile.

11) (méthylènedioxy-3,4 phényl)-2 phénylthio-2{N-[(acétyl-1 méthoxy-8 tétrahydro-1,2,3,4 quinoléinyl-5 éthyl] N-méthylamino}-5 pentanenitrile.

12) (diméthoxy-3,4 phényl)-2 (p.chlorophénylthio)-2 {N-[(acétyl-1 méthoxy-8 tétrahydro-1,2,3,4 quinoléinyl-5 éthyl] N-méthylamino}-5 pentanenitrile.

13) (diméthoxy-3,4 phényl)-2 (p.méthylphénylthio)-2 {N-[(acétyl-1 méthoxy-8 tétrahydro-1,2,3,4 quinoléinyl-5) éthyl] N-méthylamino}-5 pentanenitrile.

14) (méthoxy-3 phényl)-2 isopropyl-2{N-[(acétyl-1 méthoxy-8 tétrahydro-1,2,3,4 quinoléinyl-5) éthyl] N-méthylamino}-5 pentanenitrile.

15) (méthoxy-3 phényl)-2 dodécanyl-2(N-[acétyl-1 méthoxy-8 tétrahydro-1,2,3,4 quinoléinyl-5) éthyl] N-méthylimino}-5 pentanenitrile P.F. de l'oxalate acide : 115° C (éthanol/éther).

16) (méthoxy-3 phényl)-2 phénylthio-2 {N-[(acétyl-1 méthoxy-8 tétrahydro-1,2,3,4 quinoléinyl)-5 éthyl] N-éthylamino}-5 pentanenitrile.

17) (méthoxy-3 phényl)-2 phénylthio-2 {N-[(formyl-1 méthoxy-8 tétrahydro-1,2,3,4 quinoléinyl-5) éthyl] N-méthylamino}-5 pentanenitrile, isolé sous forme de fumarate.

18) (méthoxy-3 phényl)-2 phénylthio-2 (N-[(acétyl-1 méthoxy-8 tétrahydro-1,2,3,4 quinoléinyl-5) éthyl] N-n.propylamino}-5 pentanenitrile, isolé sous forme de fumarate.

19) (méthoxy-3 phényl)-2 phénylthio-2 {N-[(propionyl-1 méthoxy-8 tétrahydro-1,2,3,4 quinoléinyl-5) éthyl] N-méthylamino}-5 pentanenitrile, isolé sous forme de fumarate.

20) (méthoxy-3 phényl)-2 phénylsulfonyl-2 {N-[(acétyl-1 méthoxy-8 tétrahydro-1,2,3,4 quinoléinyl-5) éthyl] N-méthylamino}-5 pentanenitrile, isolé sous forme de fumarate.

21) (benzothiazolyl-6)-2 phénylthio-2{N-[(acétyl-1 méthoxy-8 tétrahydro-1,2,3,4 quinoléinyl-5) éthyl] N-méthylamino}-5 pentanenitrile, isolé sous forme de difumarate.

22) (benzoxazolyl-5)-2 phénylthio-2{N-[(acétyl-1 méthoxy-8 tétrahydro-1,2,3,4 quinoléinyl-5) éthyl] N-méthylamino}-5 pentanenitrile, isolé sous forme de difumarate.

23) (benzothiadiazol-2,1,3 yl-5)-2 phénylthio-2{N-[(acétyl-1 méthoxy-8 tétrahydro-1,2,3,4 quinoléinyl-5) éthyl] N-méthylamino}-5 pentanenitrile, isolé sous forme de difumarate.

24) méthoxy-8 quinoléinyl-5)-2 phénylthio-2{N-[(acétyl-1 méthoxy-8 tétrahydro-1,2,3,4 quinoléinyl-5) éthyl] N-méthylamino}-5 pentanenitrile, isolé sous forme de difumarate.

Exemple 25 :

(méthoxy-3 phényl)-2 phénylthio-2{N-[(acétyl-1 méthoxy-8 tétrahydro-1,2,3,4 quinoléinyl-5) éthyl] N-méthylamino}-5 pentanenitrile.

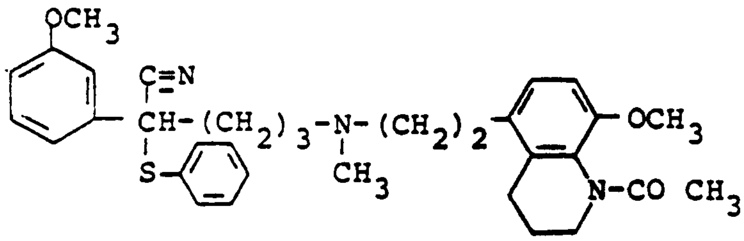

A une solution de 11,3 g de (méthoxy-3 phényl)-2 phénylthio-2 [N-méthyl N-[(méthoxy-8 quinoléinyl-5) éthyl] amino}-5 pentanenitrile (huile) préparé comme dans les exemples 1 ou 2, dans 500 ml de méthanol, on ajoute à reflux en 3 fractions égales : 3x5,6 g de Na $BH_3CN$, chaque fraction étant suivie pendant 45 minutes, d'addition de 3x89,3 ml d'HCl pour maintenir le pH à 6-7.
Après là fin de la 3ème addition d'acide, on chauffe encore 1h30, refroidit et acidifie le mélange avec 35,7 ml d'HCl concentré. On évapore à sec et extrait au $CH_2Cl_2$, lave au $CO_3Na_2$ 10%, à l'eau et sèche sur $SO_4Na_2$. On obtient 11,5 g de base brute huileuse qu'on purifie sur 300 g de silice en éluant avec le mélange ACOEt-$CH_2Cl_2$(50-50) sous 0,5 atm de $N_2$. On obtient après évaporation 6,9 g de (méthoxy-3 phényl)-2 phénylthio-2{N-méthyl N-[(méthoxy-8 tétrahydro-1,2,3,4 quinoléinyl-5) éthyl]amino}-5 pentanenitrile sous forme d'huile.
On dissout celle-ci dans 150 ml de tétrahydrofuranne anhydre, ajoute 2,03 g de triéthylamine, refroidit la solution a 5°C et coule à cette température, en 15 minutes, une solution de 1,58 g de chlorure d'acétyle dans 30 ml de tétrahydrofuranne.

On laisse 1 heure à 5°C et 1 heure a température ambiante puis filtre le chlorhydrate de triéthylamine. On évapore à sec, et obtient 5,4 g d'huile qu'on transforme en fumarate (amorphe) au sein de l'éthanol.

Les dérivés objet des exemples 5 à 17 ont également été préparés selon le procédé décrit dans l'exemple 25.

EXEMPLE 26 :

Etude pharmacologique in vitro :

A/méthodologie

a. Etude sur organes isolés prélevés chez le rat On utilise des rats Wistar mâles de 350 à 400 g. Après sacrifice rapide de l'animal, sont prélevés :

- l'aorte, disséquée et préparée en anneaux ; après une période d'équilibrage de 1 heure dans la solution physiologique de référence (1), les préparations sont stimulées par une solution riche en potassium (K+) 100mM) ou par la noradrénaline (NA) ($10^{-6}$ M).
  Les substances à tester sont alors ajoutées (prise d'essai 0,2 ml) a doses cumulées, toutes les 15 minutes.
  Les valeurs de relaxation obtenues permettent de construire une courbe

action-dose conduisant au calcul de l'IC50 en M.

- Les oreillettes droites (OD) et gauches (OG).
  OD bat spontanément :
  OG est stimulée électriquement au rythme de 3Hz (180 battements par mn) amplitude des stimulations seuil x 2).
  La stabilisation est obtenue dans le liquide physiologique (2) après 30 minutes.
  Les substances à tester sont alors ajoutées (prise d'essai 0,2 ml) à doses cumulées toutes les 15 mn.

Les effets sur OD sont chronotropes. Les effets sur OG sont inotropes. L'établissement des courbes action-dose conduisent à la mesure de l'IC50 exprimée en M.
Les caractéristiques des solutions employées sont :

(1) Solution physiologique (en M) : NaCl 112 ; KCl 5 ; KH2PO4 1,2 $CaCl_2$ 2,5 NaHCO3,25 ; glucose 11,5 Solution hyperpotassique : NaCl 17 : KCl 100 : etc... + 95% 02,5%CO2 ; pH 7,4-T° = 37°C.

(2) Solution physiologique (en mM) : NaCl 118 ; KCl 4,7 ; CaCl2 2,6 ; MgCl2 1,2 ; NaH2PO4 1 ; NaHCO3 25 ; glucose 11,1 + 95%02 ; 5%CO2 ; pH 7,4-T° =35°C.

b. Fixation sur calcium calmoduline "binding"
Les orientations de synthèse pouvant conduire à des possibilités, pour les inhibitions de la calmoduline sont évaluées par mesure de fluorescence de la calmoduline dansylée. Les substances à tester sont ajoutées dans la cuve expérimentale (2 ml). L'augmentation de fluorescence de la calmoduline dansylée permet l'établissement de courbes effet-dose ; la mesure de l'EC50 conduit à une valeur de Kd apparent. Les expériences réalisées pour différentes valeurs de concentration en calcium-calmoduline permettent, après extrapolation des valeurs de Kd apparent à O calmoduline, de déterminer les Kd caractéristiques des substances testées (méthode proposée par Johnson et Wittenauer, 1983, Biochem. 211,473-479).

c. Evaluation de la sélectivité myocarde/vaisseau
Le rapport IC50 OG/IC50 aorte conduit à une première évaluation, les valeurs élevées traduisent une sélectivité vasculaire.

d. Evaluation de la possibilité d'inhibition au niveau de la calmoduline. Le rapport Kd/IC50 aorte, lorsqu'il est voisin de 1, traduit une inhibition vasculaire à travers ce mécanisme, cette conception peut être également soutenue par une valeur relativement faible de IC50 obtenue sur l'aorte par stimulation noradrénergique.

e. De plus,

- l'artère aorte du rat sans endothélium peut être stimulée par augmentation du calcium intracellulaire de la fibre lisse par l'intermédiaire du calcium ionophore A 23187 à la concentration de $5.10^{-6}$ M. Cette contraction n'est inhibée que par des molécules ayant une action sur les mécanismes intracellulaires.
  Le vérapamil est inactif sur ce phénomène. Le produit objet de l'exemple 1 est actif avec une IC50 de l'ordre de $10^{-6}$ M. Cet effet est à rapprocher des observations de binding sur calmoduline.

B/résultats

Les résultats obtenus avec les produits illustrant l'invention sont répertoriés dans le tableau ci-après.

| PRODUITS | AORTE St : K + | AORTE St : NA | OREILLETTE DROITE (OD) EFFET CHRONOTROPE) | OREILLETTE GAUCHE (OG) (EFFET INOTROPE) | SELECTIVITE OG / AORTE (St : K + ) | Kd (cam) | Kd/ IC50 AORTE K + |
|---|---|---|---|---|---|---|---|
| EXEMPLE 1 | $7,5.10^{-7}$ | $5,5.10^{-6}$ | $10^{-5}$ | $8,2.10^{-5}$ | 100 | $8,1.10^{-7}$ | 1,08 |
| EXEMPLE 5 | $6.10^{-7}$ | $1,3.10^{-5}$ | $1,5.10^{-6}$ | $8.10^{-5}$ | 13 | $6,5.10^{-6}$ | 10,8 |
| EXEMPLE 8 | $3,6.10^{-7}$ | $3.10^{-6}$ | $2,9.10^{-6}$ | $5,5.10^{-6}$ | 15 | $2,4.10^{-6}$ | 0,66 |
| EXEMPLE 14 | $2,5.10^{-7}$ | $3.10^{-5}$ | $10^{-6}$ | $5,2.10^{-6}$ | 20 | $3,4.10^{-5}$ | 97 |
| REFERENCE VERAPAMIL | $8.10^{-7}$ | $1,4.10^{-5}$ | $2.10^{-6}$ | $2.10^{-6}$ | 5,6 | $4,5.10^{-5}$ | 195 |

C/Binding sur canaux calciques des membranes des tubules transverses du muscle squelettique :

Des mesures des constantes d'affinité pour le canal calcique montrent que l'on a un Kd exceptionnel de 1nM pour le produit de l'exemple 1 alors qu'il est de 20nM pour le vérapamil sur la même préparation.

D/Conclusions de l'étude in vitro :

Les molécules présentées conduisent à des relaxations vasculaires et à des effets inotropes et chronotropes négatifs, ces effets sont du même ordre de grandeur que ceux obtenus avec le vérapamil ; toutefois ces molécules nouvelles montrent une sélectivité vasculaire plus importante que celle présentée par le vérapamil celle-ci étant maximum avec le produit de l'exemple 1. De plus, les effets pharmacologiques des produits des 1 et 11 traduisent des mécanismes inhibiteurs intracellulaires et, en particulier, d'une inhibition de la calcium-calmoduline.

EXEMPLE 27 :

Etude pharmacologique in vivo :

A/méthodologie

Une étude hémodynamique a été réalisée sur des chiens batards de 25 à 30 kg, anesthésiés au pentobarbital sodique à la dose de 30 mg/kg i.v. intubés et ventilés, soumis à une thoracotomie au 5ème espace intercostal gauche. La mesure du débit coronaire moyen est réalisée par débimètre électromagnétique Gould SP 2202 ; la bague électromaghétique étant mise en place au niveau de la branche circonflexe de l'artère coronaire gauche. Les pressions ventriculaires gauche et aortique sont mesurées par sondes MILLAR introduites par voie artérielle fémorale. La fréquence cardiaque est mesurée par ECG Biotach Gould (dérivation D2) ; les gaz du sang aortique et du sinus coronaire sont analysés par radiomètre ABL3.

Les produits testés ont été injectés par voie veineuse fémorale.

B/résultats :

Les résultats de l'étude hémodynamique effectuée sur un certain nombre de produits représentatifs de l'invention et un produit de référence, (le vérépamil) sont regroupés dans le tableau ci-après.

VARIATIONS MAXIMALES EXPRIMEES EN Δ % QUELQUE SOIT LE TEMPS (SAUF POUR DP/DT MESUREES A 20 MINUTES)

| PRODUIT | DOSE ug/kg * IV | FC | PAM | DCM | $PO_2SC$ | A/C | dP/dt | PR |
|---|---|---|---|---|---|---|---|---|
| EXEMPLE 1 | 30 | -5 | 0 | +54 | +14 | +19 | | 0 |
| EXEMPLE 1 | 100 | -10 | -9 | +109 | +36 | +55 | 0 | +7 |
| EXEMPLE 1 | 300 | -14 | -24 | +117 | +63 | +118 | -3 | +19 |
| EXEMPLE 5 | 300 | -17 | -14 | +84 | +47 | +83 | -2 | +27 |
| EXEMPLE 8 | 300 | -7 | -16 | +77 | +36 | +52 | 0 | +15 |
| EXEMPLE 8 | 1000 | -11 | -36 | +132 | +49 | +107 | -3 | +37 |
| EXEMPLE 14 | 100 | -11 | -10 | +42 | +40 | +45 | 0 | +10 |
| EXEMPLE 14 | 300 | -15 | -23 | +84 | +59 | +99 | -6 | +32 |
| VERAPAMIL | 30 | -4 | -5 | +32 | +18 | +18 | | +6 |
| VERAPAMIL | 100 | -9 | -16 | +87 | +42 | +60 | -5 | +14 |
| VERAPAMIL | 300 | -8 | -21 | +99 | +71 | +127 | -13 | +40 |

* Les doses sont exprimées en quantité de produit sous forme de base.

FC: FREQUENCE CARDIAQUE
PAM: PRESSION ARTERIELLE MOYENNE
DCM: DEBIT CORONAIRE MOYEN
$PO_2SC$: $PO_2$ SANG SINUS CORONAIRE
A/C: RAPPORT APPORT/CONSOMMATION O2 MYOCARDIQUE
dP/dt: DERIVEE PRESSION VENTRICULAIRE GAUCHE
PR: ESPACE PR DE L'ECG

C/conclusion de l'étude in vivo :

Les mesures hémodynamiques réalisées chez le chien montrent que les produits de l'invention testés ont des propriétés au moins équivalentes à celles de la substance de référence (vérapamil) ; toutefois pour

deux d'entre elles, produits des exemples 1 et 11, l'effet cardiotrope est plus faible, traduit, en particulier, par l'absence d'effet inotrope négatif et par la diminution du pourcentage d'allongement de la durée PR.
Ces données confirment, chez l'animal, les observations réalisées sur organes isolés. Elles confortent l'aspect original des produits de l'invention comparés au vérapamil.

**Revendications**

**Revendications pour les Etats contractants suivants : BE, CH, DE, FR, GB, IT, LI, LU, NL, SE**

**1.** Les dérivés d'amino-5 pentanenitrile de formule générale I :

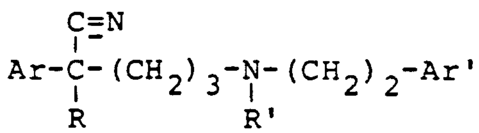

(I)

dans laquelle :

- Ar représente un radical de formule :

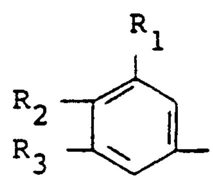

dans laquelle :

- $R_1$ représente un atome d'hydrogène ou d'halogène, un radical trifluorométhyle ou un radical alcoxy contenant de 1 à 5 atomes de carbone ; et
- $R_2$ et $R_3$, identiques ou différents représentent chacun un atome d'hydrogène ou un radical alcoxy contenant de 1 à 5 atomes de carbone ou
- $R_2$ et $R_3$ représentent ensemble un radical : $-O-CH=CH-O-$ ou $-O-CH_2-O-$ ; ou
- $R_2$ et $R_3$ forment ensemble, avec le radical phényle auquel ils sont liés un radical benzoxazolyle, benzothiazolyle, benzothiadiazolyle ou quinoléinyle ;
- R est un radical alcoyle contenant de 3 à 15 atomes de carbone en chaîne droite ou ramifiée, ou un radical de formule :

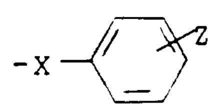

dans laquelle :

- X représente S ou $SO_2$, et
- Z représente un atome d'hydrogène, de chlore ou un radical alcoyle ou alcoxy ayant chacun de 1 à 5 atomes de carbone;
- R' représente un radical alcoyle contenant de 1 à 5 atomes de carbone; et
- Ar' représente :
  un radical de formule :

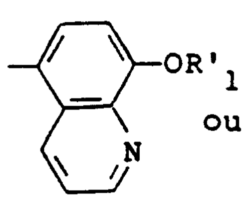

ou

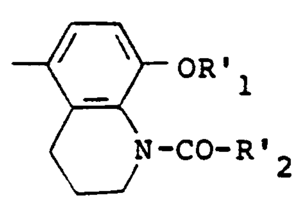

dans lesquelles :

- R'$_1$ représente un radical alcoyle contenant de 1 à 5 atomes de carbone en chaîne droite ou ramifiée, et
- R'$_2$ représente un atone d'hydrogène ou, un radical alcoyle contenant de 1 à 4 atomes de carbone en chaîne droite ou ramifiée.

**2.** Les sels physiologiquement tolérables des composés de la revendication 1 avec des acides appropriés.

**3.** Le (méthoxy-3 phényl)-2 phénylthio-2{N-[(acétyl-1 méthoxy-8 tétrahydro-1,2,3,4 quinoléinyl-5 éthyl] N-méthylamino}-5 pentanenitrile.

**4.** Le (diméthoxy-3,4 phényl)-2 isopropyl-2{N-[(acétyl-1 méthoxy-8 tétrahydro-1,2,3,4 quinoléinyl-5) éthyl] N-méthylamino}-5 pentanenitrile.

**5.** Le (diméthoxy-3,4 phényl)-2 phénylthio-2{N-[(acétyl-1 méthoxy-8 tétrahydro-1,2,3,4 quinoléinyl-5) éthyl] N-méthylamino}-5 pentanenitrile.

**6.** Le (méthoxy-3 phényl)-2 isopropyl-2{N-[acétyl-1 méthoxy-8 tétrahydro-1,2,3,4 quinoléinyl-5) éthyl] N-méthylamino}-5 pentanenitrile.

**7.** Le (méthoxy-3 phényl)-2 phénylsulfonyl-2{N-[(acétyl-1 méthoxy-8 tétrahydro-1,2,3,4 quinoléinyl-5) éthyl] N-méthylamino}-5 pentanenitrile.

**8.** Le (méthoxy-3 phényl)-2 phénylthio-2{N-[(acétyl-1 méthoxy-8 tétrahydro-1,2,3,4 quinoléinyl-5 éthyl] N-éthylamino}-5 pentanenitrile.

**9.** Le (benzothiazolyl-6)-2 phénylthio-2{N-[(acétyl-1 méthoxy-8 tétrahydro-1,2,3,4 quinoléinyl-5- éthyl] N-méthylamino]-5 pentanenitrile.

**10.** Le procédé de préparation des dérivés de la revendication 1, caractérisé en ce que l'on fait réagir un composé halogéné de formule générale II :

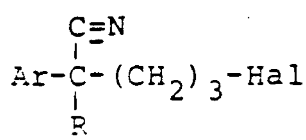

(II)

dans laquelle Ar et R ont les significations définies dans la revendication 1 et Hal représente un atome d'halogène

- avec une amine de formule générale III :

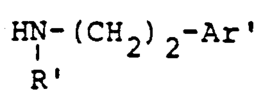

(III)

dans laquelle R' et Ar' ont les siqnifications définies dans la revendication 1; en opérant dans un solvant à une température comprise entre 60 et 100 ° C.

**11.** Le procédé de préparation des dérivés de la revendication 1, caractérisé en ce que l'on fait réagir

- un composé de formule générale IV :

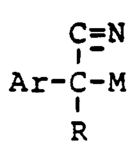

(IV)

dans laquelle Ar et R ont les significations énoncées dans la revendication 1 et M représente un atome de sodium ou de lithium,

- avec un dérivé halogéné de formule générale V :

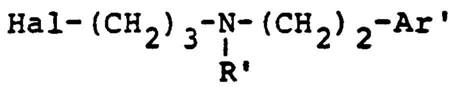

(V)

dans laquelle R' et Ar' ont les significations définies dans la revendication 1 et Hal représente un atome d'halogène; en opérant dans un solvant à une température comprise entre 60 et 110 ° C.

**12.** Le procédé de préparation des dérivés de la revendication 1 répondant à la formule générale I' :

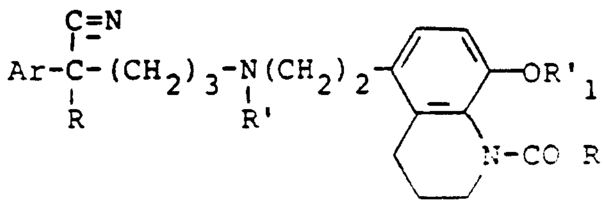

(I')

dans laquelle Ar, R, R', $R'_1$ et $R'_2$ ont les significations définies dans la revendication 1, caractérisé en ce que :

- l'on hydrogène le composé de formule générale VI :

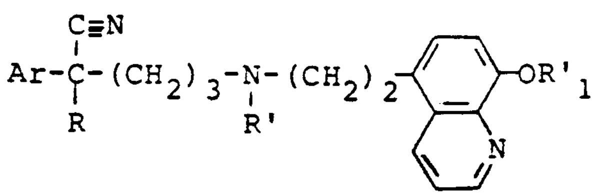

(VI)

dans laquelle Ar, R, R' et $R'_1$ ont les significations définies dans la revendication 1, et

- l'on acyle le composé ainsi obtenu de formule générale VII :

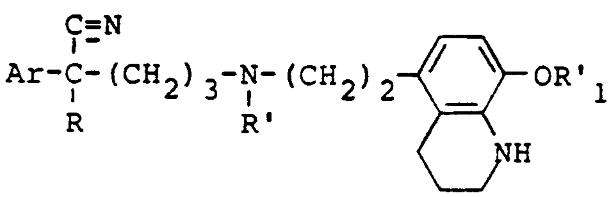

(VII)

dans laquelle Ar, R, R' et $R'_1$ ont les significations définies dans la revendication 1, avec un chlorure d'acide de formule générale VIII :

$R'_2$-CO Cl (VIII)

dans laquelle $R'_2$ a la signification énoncée dans la revendication 1.

**13.** Les compositions pharmaceutiques contenant comme principe actif un composé selon les revendications 1 à 9 avec les excipients pharmaceutiques appropriés.

**14.** Les compositions pharmaceutiques selon la revendication 13 présentées sous une forme convenant pour le traitement de maladies nécessitant des modulateurs de mouvements transmembranaires et intracellulaires du calcium.

**Revendications pour l'Etat contractant suivant : AT**

**1.** Procédé de préparation des dérivés d'amino-5 pentanenitrile de formule générale I :

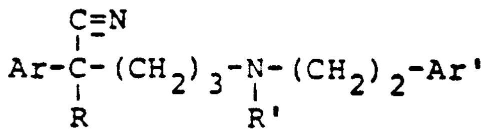

(I)

dans laquelle :

- Ar représente un radical de formule :

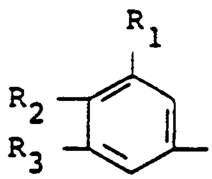

dans laquelle :

- $R_1$ représente un atome d'hydrogène ou d'halogène, un radical trifluorométhyle ou un radical alcoxy contenant de 1 à 5 atomes de carbone ; et
- $R_2$ et $R_3$, identiques ou différents représentent chacun un atome d'hydrogène ou un radical alcoxy contenant de 1 à 5 atomes de carbone ou
- $R_2$ et $R_3$ représentent ensemble un radical : $-O-CH=CH-O-$ ou $-O-CH_2-O-$ ; ou
- $R_2$ et $R_3$ forment ensemble, avec le radical phényle auquel ils sont liés un radical benzoxazolyle, benzothiazolyle, benzothiadiazolyle ou quinoléinyle;
- R est un radical alcoyle contenant de 3 à 15 atomes de carbone en chaîne droite ou ramifiée, ou un radical de formule :

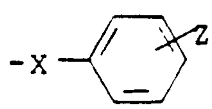

dans laquelle :

- X représente S ou $SO_2$, et
- Z représente un atome d'hydrogène, de chlore ou un radical alcoyle ou alcoxy ayant chacun de 1 à 5 atomes de carbone;
- R' représente un radical alcoyle contenant de 1 à 5 atomes de carbone; et
- Ar' représente :
  un radical de formule :

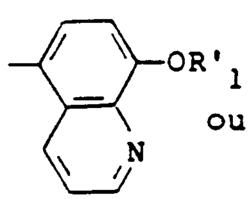

ou

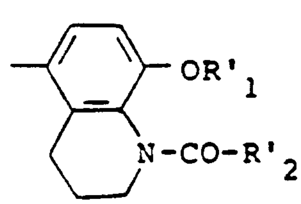

dans lesquelles :

- $R'_1$ représente un radical alcoyle contenant de 1 à 5 atomes de carbone en chaîne droite ou ramifiée, et
- $R'_2$ représente un atome d'hydrogène ou, un radical alcoyle contenant de 1 à 4 atomes de carbone en chaîne droite ou ramifiée ;

et de leurs sels d'addition avec des acides appropriés;
caractérisé en ce que l'on fait réagir :
soit :
un composé halogéné de formule générale II :

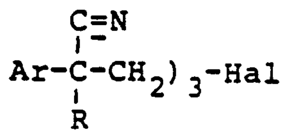

(II)

dans laquelle Ar et R ont les significations précédemment définies et Hal représente un atome d'halogène

- avec une amine de formule générale III :

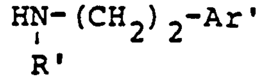

(III)

dans laquelle R' et Ar' ont les significations précédemment définies, en opérant dans un solvant à une température comprise entre 60 et 100 ° C soit

- un composé de formule générale IV :

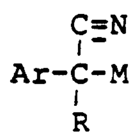

(IV)

dans laquelle Ar et R ont les significations énoncées précédemment et M représente un atome de sodium ou de lithium,

- avec un dérivé halogéné de formule générale V :

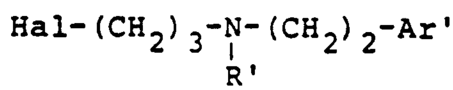

(V)

dans laquelle R' et Ar' ont les significations précédemment définies et Hal représente un atome d'halogène, en opérant dans un solvant à une température comprise entre 60 et 110 ° C, et, si on le désire, on traite les composés ainsi obtenus par des acides appropriés pour donner les sels d'addition correspondants.

**2.** Procédé de préparation des dérivés (I) de la revendication 1 répondant à la formule générale I' :

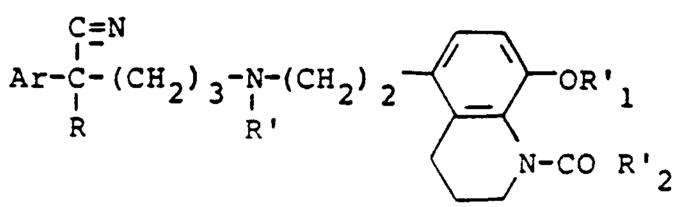

(I')

dans laquelle Ar, R, R', $R'_1$ et $R'_2$ ont les significations définies dans la revendication 1, caractérisé en ce que :

- l'on hydrogène le composé de formule générale VI :

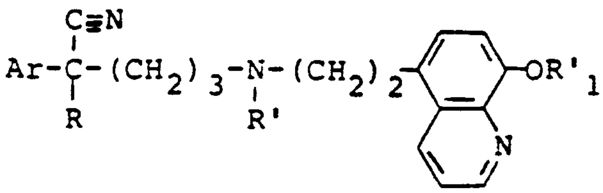

(VI)

dans laquelle Ar, R, R' et $R'_1$ ont les significations définies dans la revendication 1, et

- l'on acyle le composé ainsi obtenu de formule générale VII :

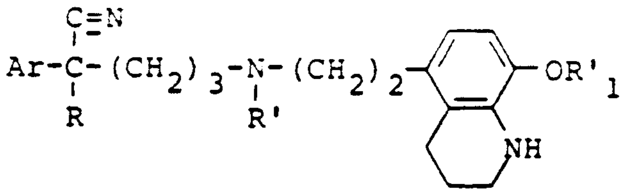

(VII)

dans laquelle Ar, R, R' et $R'_1$ ont les significations définies dans la revendication 1, avec un chlorure d'acide de formule générale VIII :

$R'_2$-CO Cl (VIII)

dans laquelle $R'_2$ a la signification énoncée dans la revendication 1;
et, si on le désire on traite les composés ainsi obtenus par des acides appropriés pour donner les sels d'addition correspondants.

**Claims**

**Claims for the following Contracting States : BE, CH, DE, FR, GB, IT, LI, LU, NL, SE**

**1.** 5-aminopentanenitrile derivatives of the general formula I:

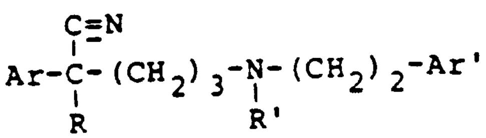

(I)

in which:

- Ar represents a radical of the formula:

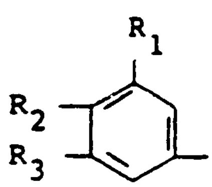

in which:

- $R_1$ represents a hydrogen or halogen atom, a trifluoromethyl radical or an alkoxy radical containing from 1 to 5 carbon atoms; and
- $R_2$ and $R_3$, which are identical or different, each represents a hydrogen atom or an alkoxy radical containing from 1 to 5 carbon atoms, or
- $R_2$ and $R_3$ together represent a radical -O-CH = CH-O- or -O-$CH_2$-O-, or
- $R_2$ and $R_3$, with the phenyl radical to which they are bonded, together form a benzoxazolyl, benzothiazolyl, benzothiadiazolyl or quinolyl radical;

- R is a straight-chain or branched alkyl radical containing from 3 to 15 carbon atoms, or a radical of the formula:

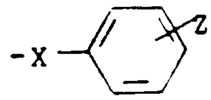

in which:
- X represents S or $SO_2$, and
- Z represents a hydrogen or chlorine atom or an alkyl or alkoxy radical each having from 1 to 5 carbon atoms;
- R' represents an alkyl radical containing from 1 to 5 carbon atoms; and
- Ar' represents:
  a radical of the formula:

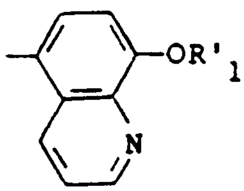
or
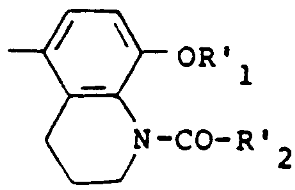

in which:
- $R'_1$ represents a straight-chain or branched alkyl radical containing from 1 to 5 carbon atoms, and
- $R'_2$ represents a hydrogen atom or a straight-chain or branched alkyl radical containing from 1 to 4 carbon atoms.

**2.** The physiologically tolerable salts of the compounds of claim 1 with suitable acids.

**3.** 2-(3-methoxyphenyl)-2-phenylthio-5-(N-[(1-acetyl-8-methoxy-1,2,3,4-tetrahydroquinol-5-yl)ethyl]-N-methylamino)-pentanenitrile.

**4.** 2-(3,4-dimethoxyphenyl)-2-isopropyl-5-(N-[(1-acetyl-8-methoxy-1,2,3,4-tetrahydroquinol-5-yl)ethyl]-N-methylamino)-pentanenitrile.

**5.** 2-(3,4-dimethoxyphenyl)-2-phenylthio-5-[N-[(1-acetyl-8-methoxy-1,2,3,4-tetrahydroquinol-5-yl)ethyl]-N-methyl amino)-pentanenitrile.

**6.** 2-(3-methoxyphenyl)-2-isopropyl-5-(N-[(1-acetyl-8-methoxy-1,2,3,4-tetrahydroquinol-5-yl)ethyl]-N-methylamino)-pentanenitrile.

**7.** 2-(3-methoxyphenyl)-2-phenylsulphonyl-5-(N-[(1-acetyl-8-methoxy-1,2,3,4-tetrahydroquinol-5-yl)ethyl]-N-methylamino)-pentanenitrile.

**8.** 2-(3-methoxyphenyl)-2-phenylthio-5-(N-[(1-acetyl-8-methoxy-1,2,3,4-tetrahydroquinol-5-yl)ethyl]-N-ethylamino)-pentanenitrile.

**9.** 2-(6-benzothiazolyl)-2-phenylthio-5-(N-[(1-acetyl-8-methoxy-1,2,3,4-tetrahydroquinol-5-yl)ethyl]-N-methylamino)-pentanenitrile.

**10.** Process for the preparation of the derivatives of claim 1, characterised in that
- a halogenated compound of the general formula II:

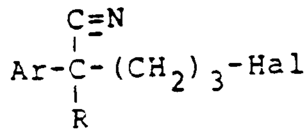

(II),

in which Ar and R have the meanings defined in claim 1 and Hal represents a halogen atom, is reacted

- with an amine of the general formula III:

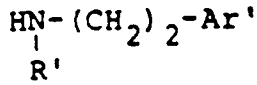

(III)

in which R' and Ar' have the meanings defined in claim 1, the reaction being carried out in a solvent at a temperature of from 60 to 100°C.

**11.** Process for the preparation of the derivatives of claim 1, characterised in that

- a compound of the general formula IV:

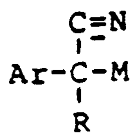

(IV),

in which Ar and R have the meanings given in claim 1 and M represents a sodium or lithium atom, is reacted

- with a halogenated derivative of the general formula V:

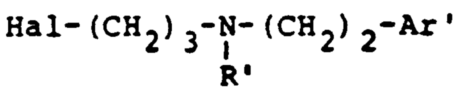

(V)

in which R' and Ar' have the meanings defined in claim 1 and Hal represents a halogen atom, the reaction being carried out in a solvent at a temperature of from 60 to 110°C.

**12.** Process for the preparation of the derivatives of claim 1 corresponding to the general formula I':

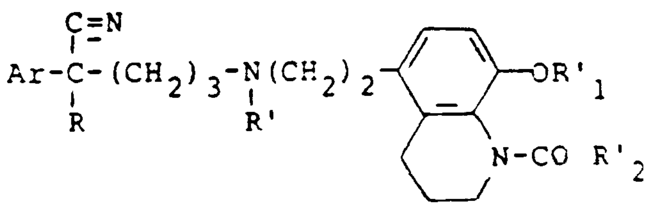

(I')

in which Ar, R, R', $R'_1$ and $R'_2$ have the meanings defined in claim 1, characterised in that:

- the compound of the general formula VI:

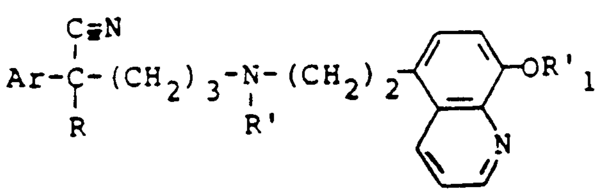

(VI),

in which Ar, R, R' and $R'_1$ have the meanings defined in claim 1, is hydrogenated, and

- the resulting compound of the general formula VII:

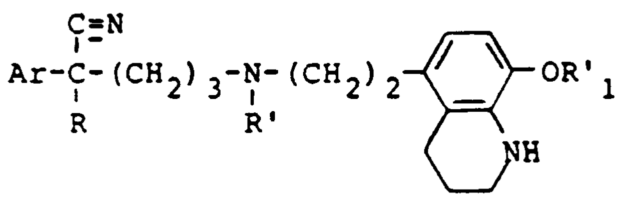

(VII),

in which Ar, R, R' and $R'_1$ have the meanings defined in claim 1, is acylated with an acid chloride of the general formula VIII:

$R'_2$-COCl (VIII)

in which $R'_2$ has the meaning given in claim 1.

**13.** Pharmaceutical compositions containing as active ingredient a compound according to claims 1 to 9 with suitable pharmaceutical carriers.

**14.** Pharmaceutical compositions according to claim 13, presented in a form suitable for the treatment of disorders requiring modulators of transmembrane and intracellular movements of calcium.

**Claims for the following Contracting State : AT**

**1.** Process for the preparation of the 5-aminopentanenitrile derivatives of the general formula I:

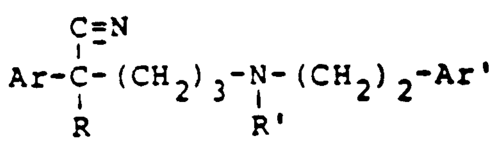

(I)

in which:

- Ar represents a radical of the formula:

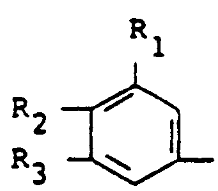

in which:

- $R_1$ represents a hydrogen or halogen atom, a trifluoromethyl radical or an alkoxy radical containing from 1 to 5 carbon atoms; and
- $R_2$ and $R_3$, which are identical or different, each represents a hydrogen atom or an alkoxy radical containing from 1 to 5 carbon atoms, or
- $R_2$ and $R_3$ together represent a radical -O-CH=CH-O- or -O-$CH_2$-O-, or
- $R_2$ and $R_3$, with the phenyl radical to which they are bonded, together form a benzoxazolyl, benzothiazolyl, benzothiadiazolyl or quinolyl radical;
- R is a straight-chain or branched alkyl radical containing from 3 to 15 carbon atoms, or a radical of the formula:

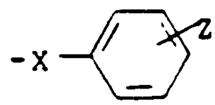

in which:

- X represents S or $SO_2$, and
- Z represents a hydrogen or chlorine atom or an alkyl or alkoxy radical each having from 1 to 5 carbon atoms;
- R' represents an alkyl radical containing from 1 to 5 carbon atoms; and
- Ar' represents: a radical of the formula:

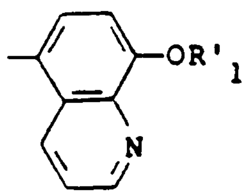

or

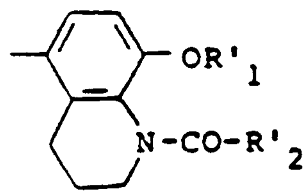

in which:

- $R'_1$ represents a straight-chain or branched alkyl radical containing from 1 to 5 carbon atoms, and
- $R'_2$ represents a hydrogen atom or a straight-chain or branched alkyl radical containing from 1 to 4 carbon atoms,

and their addition salts with suitable acids, characterised in that:

either

- a halogenated compound of the general formula II:

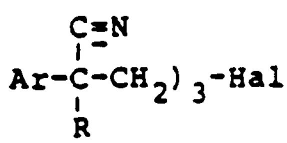

(II),

in which Ar and R have the meanings defined above and Hal represents a halogen atom, is reacted

- with an amine of she general formula III:

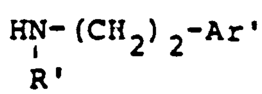

(III)

in which R' and Ar' have the meanings defined above, the reaction being carried out in a solvent at a temperature of from 60 to 100 ° C,

or

- a compound of the general formula IV:

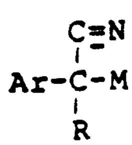

(IV),

in which Ar and R have the meanings given above and M represents a sodium or lithium atom, is reacted

- with a halogenated derivative of the general formula V:

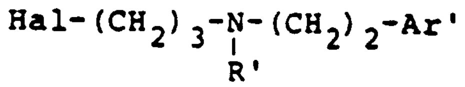

(V)

in which R' and Ar' have the meanings defined above and Hal represents a halogen atom, the reaction being carried out in a solvent at a temperature of from 60 to 110 °C,

and, if desired, the resulting compounds are treated with suitable acids to give the corresponding addition salts.

**2.** Process for the preparation of the derivatives (I) of claim 1 corresponding to the general formula I':

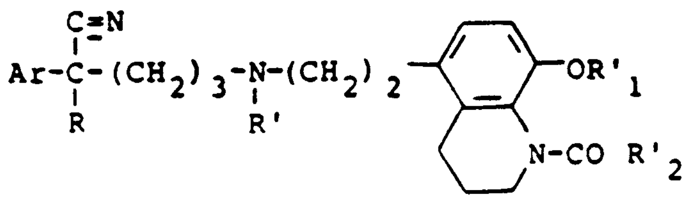

(I')

in which Ar, R, R', $R'_1$ and $R'_2$ have the meanings defined in claim 1, characterised in that:

- the compound of the general formula VI:

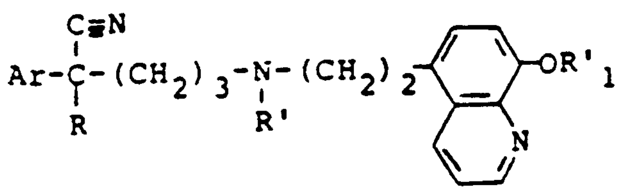

(VI),

in which Ar, R, R' and $R'_1$ have the meanings defined in claim 1, is hydrogenated, and

- the resulting compound of the general formula VII:

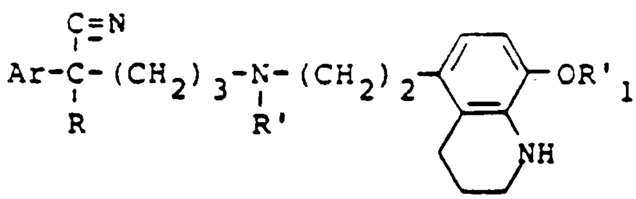

(VII),

in which Ar, R, R' and $R'_1$ have the meanings defined in claim 1, is acylated with an acid chloride of the general formula VIII:

$R'_2$-COCl (VIII)

in which $R'_2$ has the meaning given in claim 1,

and, if desired, the resulting compounds are treated with suitable acids to give the corresponding addition salts.

**Patentansprüche**
**Patentansprüche für folgende Vertragsstaaten : BE, CH, DE, FR, GB, IT, LI, LU, NL, SE**

**1.** 5-Amino-pentannitril-Derivate der allgemeinen Formel I

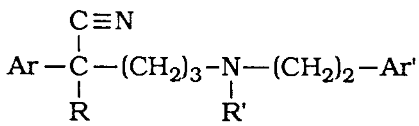

(I)

in der

- Ar eine Gruppe der Formel

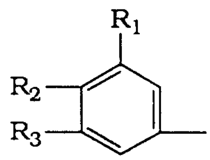

in der

- $R_1$ ein Wasserstoffatom, ein Halogenatom, eine Trifluormethylgruppe oder eine Alkoxygruppe mit 1 bis 5 Kohlenstoffatomen und
- $R_2$ und $R_3$, die gleichartig oder verschieden sein können, jeweils Wasserstoffatome oder Alkoxygruppen mit 1 bis 5 Kohlenstoffatomen oder
- $R_2$ und $R_3$ gemeinsam eine Gruppe der Formel
- O - CH = CH - O - oder - O - $CH_2$ - O -; oder
- $R_2$ und $R_3$ gemeinsam mit dem Phenylrest, an den sie gebunden sind, eine Benzoxazolylgruppe, eine Benzothiazolylgruppe, eine Benzothladiazolylgruppe oder eine Chinolinylgruppe darstellen;
- R eine geradkettlge oder verzweigte Alkylgruppe mit 3 bis 15 Kohlenstoffatomen oder eine Gruppe der Formel

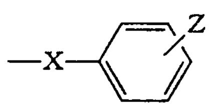

in der

- X S oder $SO_2$ und
- Z ein Wasserstoffatom, ein Chloratom oder eine Alkyl- oder Alkoxygruppe mit 1 bis 5 Kohlenstoffatomen darstellen;
- R' eine Alkylgruppe mit 1 bis 5 Kohlenstoffatomen; und
- Ar' eine Gruppe der Formel

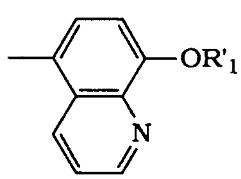

oder

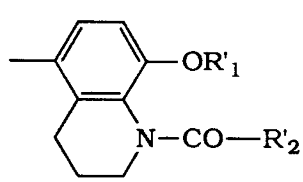

worin

- $R'_1$ eine geradkettige oder verzweigte Alkylgruppe mit 1 bis 5 Kohlenstoffatomen und
- $R'_2$ ein Wasserstoffatom oder eine geradkettige oder verzweigte Alkylgruppe mit 1 bis 4 Kohlenstoffatomen darstellen,

bedeuten.

**2.** Die physiologisch verträglichen Salze der Verbindungen nach Anspruch 1 mit geeigneten Säuren.

**3.** 2-(3-Methoxyphenyl)-2-phenylthio-5-{N-[(1-acetyl-8-methoxy-1,2,3,4-tetrahydro-chinolin-5-yl)-ethyl]-N-methylamino}-pentannitril.

**4.** 2-(3,4-Dimethoxy-phenyl)-2-isopropyl-5-{N-[(1-acetyl-8-methoxy-1,2,3,4-tetrahydro-chinolin-5-yl)-ethyl]-N-methylamino}-pentannitril.

**5.** 2-(3,4-Dimethoxy-phenyl)-2-phenylthio-5-[N[(1-acetyl-8-methoxy-1,2,3,4-tetrahydro-chinolin-5-yl)-ethyl]-N-methylamino}-pentannitril.

**6.** 2-(3-Methoxy-phenyl)-2-isopropyl-5-{N-[(1-acetyl-8-methoxy-1,2,3,4-tetrahydro-chinolin-5-yl)-ethyl]-N-methylamino}-pentannitril.

**7.** 2-(3-Methoxy-phenyl)-2-phenylsulfonyl-5-{N-[(1-acetyl-8-methoxy-1,2,3,4-tetrahydro-chinolin-5-yl)-ethyl]-N-methylamino}-pentannitril.

**8.** 2-(3-Methoxy-phenyl)-2-phenylthio-5-{N-[(1-acetyl-8-methoxy-1,2,3,4-tetrahydro-chinolin-5-yl)-ethyl]-N-ethylamino}-pentannitril.

**9.** 2-(Benzothiazol-6-yl)-2-phenylthio-5-[N-[(1-acetyl-8-methoxy-1,2,3,4-tetrahydro-chinolin-5-yl)-ethyl]-N-methylamino]-pentannitril.

**10.** Verfahren zur Herstellung der Derivate nach Anspruch 1, **dadurch gekennzeichnet**, daß man eine Halogenverbindung der allgemeinen Formel II

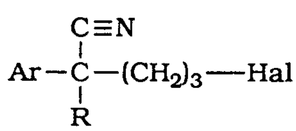

(II)

in der Ar und R die in Anspruch 1 angegebenen Bedeutungen besitzen und Hal ein Halogenatom darstellt,

- mit einem Amin der allgemeinen Formel III

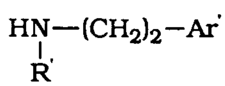

(III)

in der R' und Ar' die in Anspruch 1 angegebenen Bedeutungen besitzen, in einem Lösungsmittel bei einer Temperatur zwischen 60 und 100 ° C umsetzt.

**11.** Verfahren zur Herstellung der Derivate nach Anspruch 1, **dadurch gekennzeichnet**, daß man

- eine Verbindung der allgemeinen Formel IV

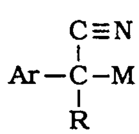

(IV)

in der Ar und R die in Anspruch 1 angegebenen Bedeutungen besitzen und M ein Natrium- oder Lithiumatom darstellt,

- mit einem Halogenderivat der allgemeinen Formel V

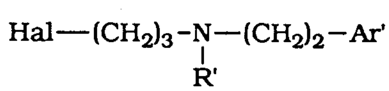

(V)

in der R' und Ar' die in Anspruch 1 angegebenen Bedeutungen besitzen und Hal ein Halogenatom

darstellt, in einem Lösungsmittel bei einer Temperatur zwischen 60 und 110 ° C umsetzt.

**12.** Verfahren zur Herstellung der Derivate nach Anspruch 1 der allgemeinen Formel I'

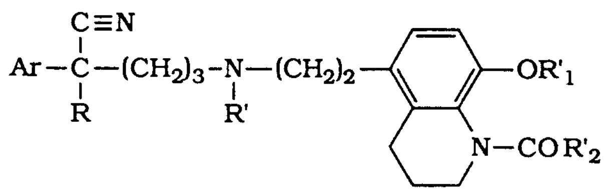

(I')

in der Ar, R, R', $R'_1$ und $R'_2$ die in Anspruch 1 angegebenen Bedeutungen besitzen, **dadurch gekennzeichnet**, daß man

- die Verbindung der allgemeinen Formel VI

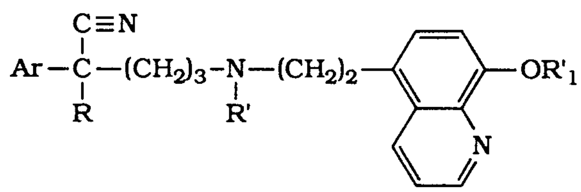

(VI)

in der Ar, R, R' und $R'_1$ die in Anspruch 1 angegebenen Bedeutungen besitzen, hydriert, und

- die in dieser Weise erhaltene Verbindung der allgemeinen Formel VII

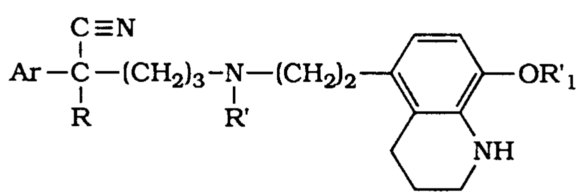

(VII)

in der Ar, R, R' und $R'_1$ die in Anspruch 1 angegebenen Bedeutungen besitzen, mit einem Säurechlorid der allgemeinen Formel VIII

$R'_2$ - CO Cl (VIII)

in der $R'_2$ die in Anspruch 1 angegebenen Bedeutungen besitzt, acyliert.

**13.** Pharmazeutische Zubereitungen enthaltend als Wirkstoff eine Verbindung nach einem der Ansprüche 1 bis 9 zusammen mit geeigneten pharmazeutischen Trägermaterialien.

**14.** Pharmazeutische Zubereitungen nach Anspruch 13 in einer Form, die zur Behandlung von Erkrankungen, die Modulatoren der transmembranen und intrazellulären Bewegung von Calcium erforderlich machen, geeignet ist.

**Patentansprüche für folgenden Vertragsstaat : AT**

**1.** Verfahren zur Herstellung von 5-Amino-pentannitril-Derivaten der allgemeinen Formel I

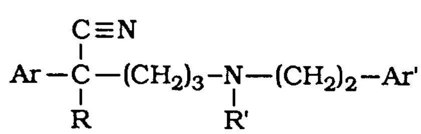

(I)

in der

- Ar eine Gruppe der Formel

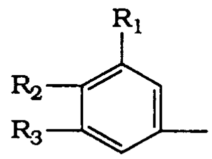

in der

- $R_1$ ein Wasserstoffatom, ein Halogenatom, eine Trifluormethylgruppe oder eine Alkoxygruppe mit 1 bis 5 Kohlenstoffatomen und
- $R_2$ und $R_3$, die gleichartig oder verschieden sein können, jeweils Wasserstoffatome oder Alkoxygruppen mit 1 bis 5 Kohlenstoffatomen oder
- $R_2$ und $R_3$ gemeinsam eine Gruppe der Formel
- O - CH = CH - O - oder - O - $CH_2$ - O -; oder
- $R_2$ und $R_3$ gemeinsammit dem Phenylrest, an den sie gebunden sind, eine Benzoxazolylgruppe, eine Benzothiazolylgruppe, eine Benzothiadiazolylgruppe oder eine Chinolinylgruppe darstellen;
- R eine geradkettige oder verzweigte Alkylgruppe mit 3 bis 15 Kohlenstoffatomen oder eine Gruppe der Formel

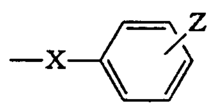

in der

- X S oder $SO_2$ und
- Z ein Wasserstoffatom, ein Chloratom oder eine Alkyl- oder Alkoxygruppe mit 1 bis 5 Kohlenstoffatomen darstellen;
- R' eine Alkylgruppe mit 1 bis 5 Kohlenstoffatomen; und
- Ar' eine Gruppe der Formel

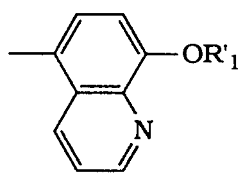

oder

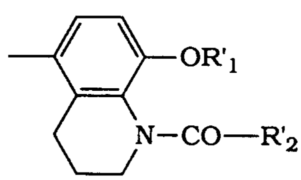

worin

- $R'_1$ eine geradkettige oder verzweigte Alkylgruppe mit 1 bis 5 Kohlenstoffatomen und
- $R'_2$ ein Wasserstoffatom oder eine geradkettige oder verzweigte Alkylgruppe mit 1 bis 4 Kohlenstoffatomen darstellen,

bedeuten,

und deren Additionssalze mit geeigneten Säuren, **dadurch gekennzeichnet**, daß man entweder eine Halogenverbindung der allgemeinen Formel II

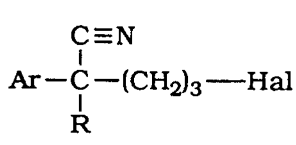

(II)

in der Ar und R die oben angegebenen Bedeutungen besitzen und Hal ein Halogenatom darstellt,

- mit einem Amin der allgemeinen Formel III

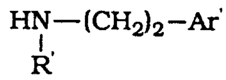

(III)

in der R' und Ar' die oben angegebenen Bedeutungen besitzen, in einem Lösungsmittel bei einer Temperatur zwischen 60 und 100 ° C umsetzt, oder

- eine Verbindung der allgemeinen Formel IV

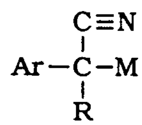

(IV)

in der Ar und R die oben angegebenen Bedeutungen besitzen und M ein Natrium- oder ein Lithiumatom darstellt,

- mit einem Halogenderivat der allgemeinen Formel V

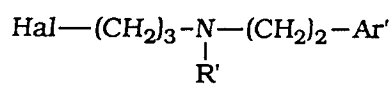

(V)

in der R' und Ar' die oben angegebenen Bedeutungen besitzen und Hal ein Halogenatom darstellt, in einem Lösungsmittel bei einer Temperatur zwischen 60 und 110 ° C umsetzt und gewünschtenfalls die in dieser Weise erhaltenen Verbindungen mit geeigneten Säuren in die entsprechenden Additionssalze überführt,

**2.** Verfahren zur Herstellung der Derivate der allgemeinen Formel I nach Anspruch 1 der allgemeinen Formel I'

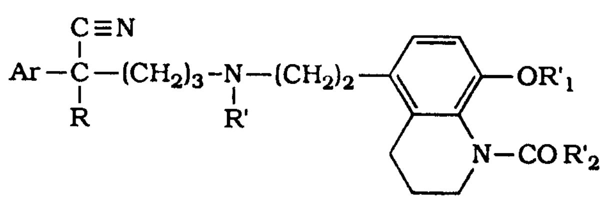

(I')

in der Ar, R, R', $R'_1$ und $R'_2$ die in Anspruch 1 angegebenen Bedeutungen besitzen, **dadurch gekennzeichnet**, daß man

- die Verbindung der allgemeinen Formel VI

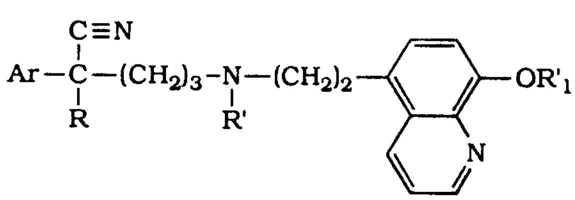

(VI)

in der Ar, R, R' und $R'_1$ die in Anspruch 1 angegebenen Bedeutungen besitzen, hydriert und

- die in dieser Weise erhaltene Verbindung der allgemeinen Formel VII

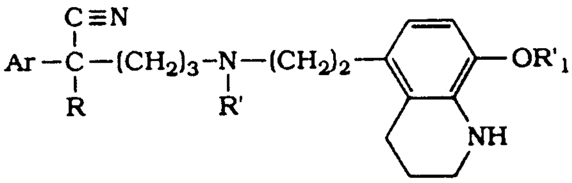

(VII)

in der Ar, R, R' und $R'_1$ die in Anspruch 1 angegebenen Bedeutungen besitzen, mit einem Säurechlorid der allgemeinen Formel VIII

$R'_2$ - CO Cl (VIII)

in der $R'_2$ die in Anspruch 1 angegebenen Bedeutungen besitzt, acyliert; und gewünschtenfalls die in dieser Weise erhaltenen Verbindungen mit geeigneten Säuren in die entsprechenden Additionssalze überführt.